# EUROPEAN PATENT APPLICATION

(11) **EP 1 506 964 A1**
(43) Date of publication of application: **16.02.2005**
(21) Application number: 03018503.7
(22) Date of filing: 15.08.2003
(51) Int. Cl.: C07D 327/04, C08G 75/14

(54) **Cyclic dithiocarbonates, their preparation and application**

(71) Applicant: Henkel Kommanditgesellschaft auf Aktien, 40589 Düsseldorf (DE)
(72) Inventor: Motokucho, Suguru, Iizuka-shi, Fukuoka (JP); Sudo, Atsushi, Kaho-gun, Fukuoka (JP); Endo, Takeshi, Yamagata 992-8510 (JP); Itagaki, Yoshiteru, Ehime 791-8005 (JP); Kaneko, Ryosuke, Kanagawa 248-0025 (JP); Uenishi, Kazuya, Kaho-gun, Fukuoka (JP); Karim, Sikder Mohammad Abdul, Fukuoka 820-0011 (JP)
(74) Representative: Leifert & Steffan

(57) **Abstract**

The present invention relates to cyclic 5-membered ring dithiocarbonate compounds of the general formula 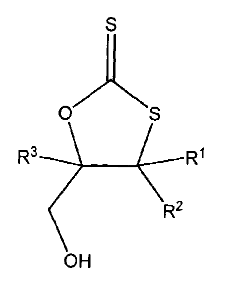 wherein, in the formula, R¹, R², and R³ are the same or different, each of which denotes hydrogen or a C1 to C4 straight-chain or branched alkyl. The invention further relates to a process for producing these compounds and a variety of products obtainable by reacting the title compounds.

## Description

The present invention relates to cyclic 5-membered ring dithiocarbonate compounds, to a process for producing the same and a variety of products obtainable by reacting the title key intermediate compounds.

Various 5-membered cyclic dithiocarbonates (1,3-oxathiolane-2-thiones) have been synthesized and applied as monomers to polymer synthesis. Compounds and polymers comprising dithiocarbonate groups have various uses. For example, they are used as ingredients for coatings, adhesives, inks, sealing agents or the like. Their physical properties as hardness, strength, adhesion, water resistance, chemical resistance, heat resistance, and the like can be improved by allowing cross-linking reactions to occur under specific conditions after application or printing of the compositions.

US-B-6 372 871 discloses a composition, comprising a polymer or a compound having at least one 5-membered ring dithiocarbonate group. Besides other polymers the synthesis of a polymeric molecule having both dithiocarbonate and hydrophilic groups by radical co-polymerization of a methacrylate monomer having dithiocarbonate with an appropriate co-monomer is described. As a candidate co-monomer 2-hydroxyethylmethacrylate is proposed. Further the reaction of an oligomeric bisphenol A-epichlorohydrin adduct with carbon disulfide is disclosed. The corresponding product should have dithiocarbonate moieties and hydroxyl moieties, which are originated from the used bisphenol A-epichlorohydrin adduct, however the product is a mixture of oligomers having structural ambiguity.

Kihara et al., J. Org. Chem. 1995, 60, 473-475 describe the preparation of 1,3-oxathiolane-2-thiones by the reaction of oxirane and carbon disulfide.

However, there has been no monomer available so far having dithiocarbonate moiety and hydroxyl group in 1:1 ratio.

It is an object of the present invention is to provide new dithiocarbonate compounds having one dithiocarbonate group and one hydroxy group per molecule which are valuable intermediate compounds for various uses. A further object of the present is to provide a process for producing the dithiocarbonate compounds of the invention. Another aspect of the present invention is to provide uses of the compounds of the invention, e.g. in the manufacture of adhesives, coating and sealing compositions or optical or photoresist materials.

The present invention provides cyclic 5-membered ring dithiocarbonate compounds of the general formula 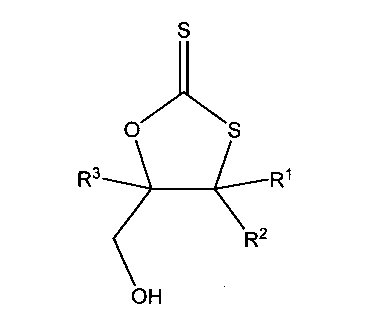 wherein, in the formula, R¹, R² and R³ are the same or different, each of which denotes hydrogen or a C1 to C4 straight-chain or branched alkyl (in the following referred to as DTC-OH independent of possible variations in R¹, R² and R³, if not specified otherwise, e.g. by reference to a specific scheme).

In the above formula preferably R¹, R², and R³ denote hydrogen.

The dithiocarbonate compounds of the present invention are highly versatile precursors to synthesize various monomers having a dithiocarbonate structure. They can be reacted with (activated) carboxylic acids or isocyanates as shown in the following diagram:

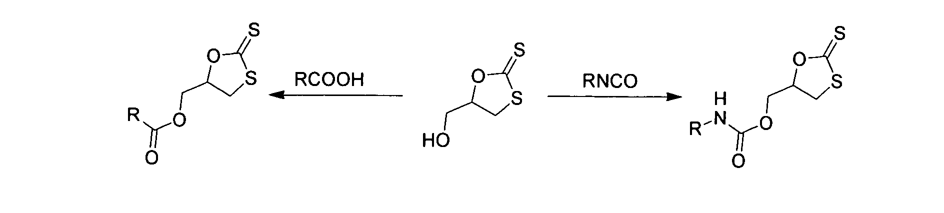

The resulting compounds which are also compounds of the present invention, are highly useful in a variety of applications.

Even though the preceding diagram shows a reaction of monocarboxylic acids and monoisocyanates, the reaction is not limited thereto, since (activated) mono-, di- and polycarboxylic acids and mono-, di- or polyisocyanates can be employed.

As activated carboxylic acids used for modification of DTC-OH in the above reaction diagram e.g. acid chlorides, activated esters, acid anhydrides or lactones as any other known activated carboxylic acid derivatives can be employed.

The following diagram shows the reaction with carboxylic acid chlorides:

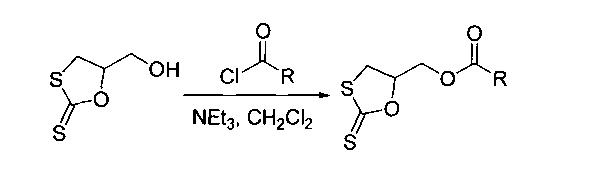

Any carboxylic acid chloride can be employed in the reaction. Examples range from fatty acid chlorides like acetic acid chloride, unsaturated acid chlorides like acrylic acid chlorides and methacrylic acid chlorides to carboxy arylic or carboxy aralkylic compounds like benzoyl chloride. Acids with two or more carboxylic acid chloride groups can also be employed.

The reactions as performed with carboxylic acid chlorides can also be carried out with the corresponding anhydrides and lactones like caprolactone and diketene (i.e. vinylaceto-β-lactone) as shown in the following general diagram:

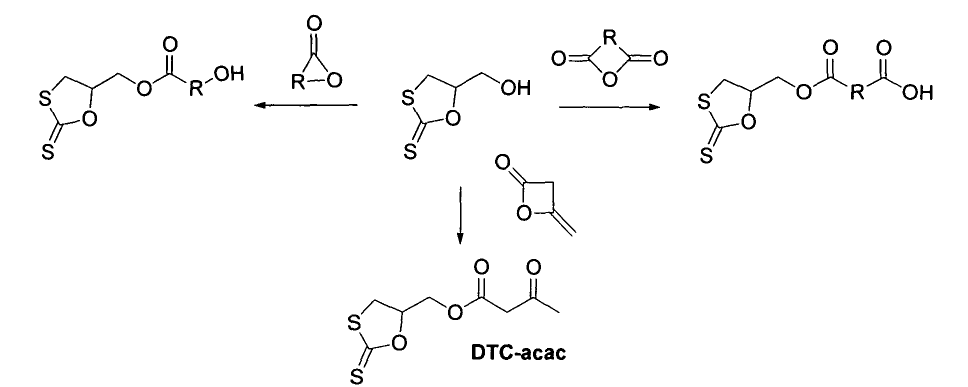

Mixed reactions of DTC-OH with diacid chlorides in the presence of diols to produce polyesters with terminal DTC groups can similarly be carried out according to the following diagram:

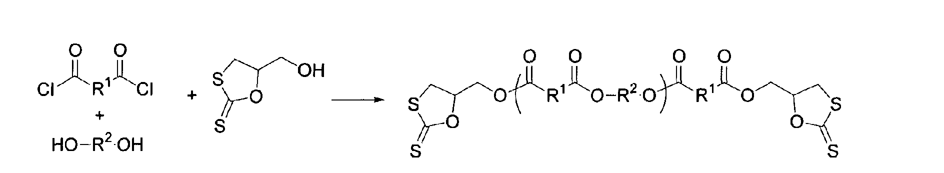

It is possible to perform the reaction as a single-step reaction or alternatively a reaction between the diol and the dichloride can be performed first, followed by the reaction with DTC-OH.

Since the DTC group is highly reactive with amino groups and also undergoes cationic polymerization by any alkyl triflates such as methyl trifluoromethanesulfonate, any protonic acids such as trifluoromethanesulfonic acid, any Lewis acids such as tin tetrachloride, any thermally latent initiators such as benzylsulfonium hexafluoroantimonate, and any photo latent initiators such as diaryliodonium salt, but not affected by moisture, such polyesters having DTC groups at the terminal positions are highly desired products for use in water-emulsion adhesives, paints, coatings and the like.

The use of diisocyanates in the preceding diagram, instead of the diacid chlorides, leads to the corresponding polyurethanes with similar fields of application. The dithiocarbonate monomers having a urethane moiety (obtained by reaction with isocyanate) are especially useful as expandable monomers, e.g. in epoxy-amine curing reactions wherein volume shrinkage is reduced. Moreover adhesion strength can also be improved by the addition of the derivative monomers, especially when the monomers have two or more dithiocarbonate groups in the molecule.

In the following diagram the wavy line stands for either polymer chains like polyesters, polyurethanes and mixed polyester/polyurethanes or for monomeric spacers of any nature ranging from (hetero)aliphatic, (hetero)cycloaliphatic, (hetero)aromatic to (hetero)araliphatic residues:

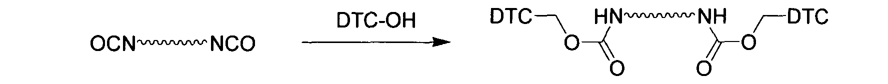

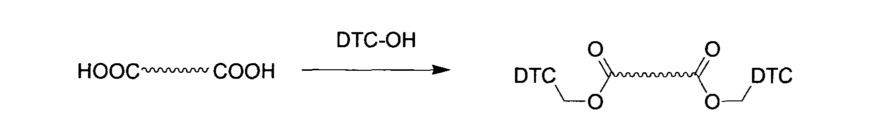

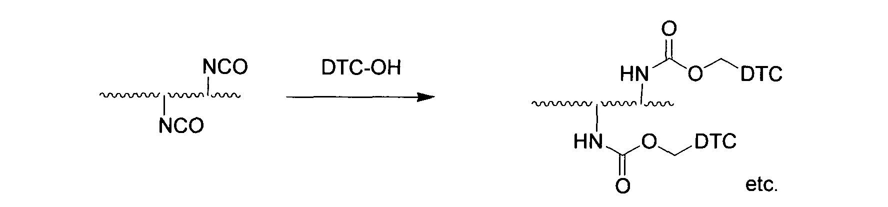

As shown for isocyanates in the last reaction equation, a modification of reactive side chains in polymers is also possible.

It is e.g. possible to react a DTC-OH of the present invention with an isocyanate having the general formula: 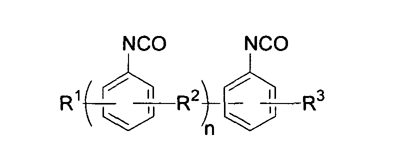 wherein, n>0; the benzene rings are optionally substituted by further reactive and/or non-reactive functional groups and/or fused with a further aromatic ring; R¹, R² and R³ can be attached at any position of the benzene ring or fused benzene ring; R¹ and R³ can be same or different and denote hydrogen, (hetero)alkyl, (hetero)aromatic and halogen; and R² is a (hetero)alkylene residue, a (hetero)aromatic residue, oxygen, sulfur, oxidized sulfur, a carbonyl residue, a substituted silicone residue, or any polymer chain, like a polyester or a polyurethane.

Japanese Patent Publication No. 07-145164 discloses a limited number of compounds bearing one cyclic dithiocarbonate group at each end of a molecule, whereby those end groups are essentially spaced by an alkylene or phenylene group. The compounds described in said Japanese Patent Publication were prepared from the corresponding epoxy precursors by cycloaddition of carbon disulfide, which significantly limits the number of the possible available compounds. It was assumed that those molecules might be useful as cross-linking agents, but actual feasibility studies were not shown.

Other reactions of the hydroxyl group, addition to vinyl ether, condensation with siloxy compound, etc. are also possible to obtain various dithiocarbonate monomers.

Analogous reactions can be applied to modify various other polymers apart from polyurethanes and polyesters. The presence of any end groups or side chains, which are reactive with hydroxyl group, is usually sufficient to obtain highly reactive but moisture-resistant polymers for water emulsion adhesives or coating materials.

The dithiocarbonate compounds of the present invention are not only valuable intermediates which can be bound to suitable polymers by reacting the hydroxy group for example with carboxyl groups or isocyanate groups of the polymer in order to incorporate dithiocarbonate groups into the polymer as described above, since the dithiocarbonate moiety can be selectively reacted before the modification of hydroxyl group.

DTC-OH can e.g. be reacted with compounds having two or more amino groups in order to produce useful components of adhesives, coatings, and sealing materials.

Finally they can be polymerized in order to produce high sulfur-containing polymers which can be used as optical materials, for removing heavy metal compounds from waste water and as a photoresist materials.

Therefore the present invention also provides a polymer having thiourethane and disulfide bonds in the main chain and hydroxy groups in the side chains obtainable by reacting the cyclic 5-membered ring dithiocarbonate compounds of claim 1 having the general formula 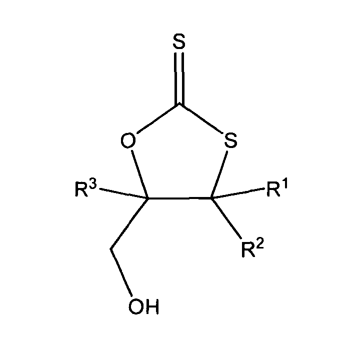 wherein, in the formula, R¹, R², and R³ are the same or different, each of which denotes hydrogen or a C1 to C4 straight-chain or branched alkyl with a compounds having two or more amino groups in an organic solvent and reacting the resultant intermediate compound with oxygen.

Due to the hydrophilicity of the hydroxyl groups, the polymers are soluble in alcohols and miscible in water. Such solubility and miscibility has not been accomplished by using previously known cyclic dithiocarbonates.

In a further step the pendant hydroxy groups of this polymer can be modified by its reaction with carboxylic acid derivatives such as acyl halides (e.g. acyl chlorides), lactones, and acid anhydrides, isocyanates, silyl compounds (e.g. silyl chlorides) or vinyl ether.

One process to obtain those compounds of the invention is shown by the following general diagram:

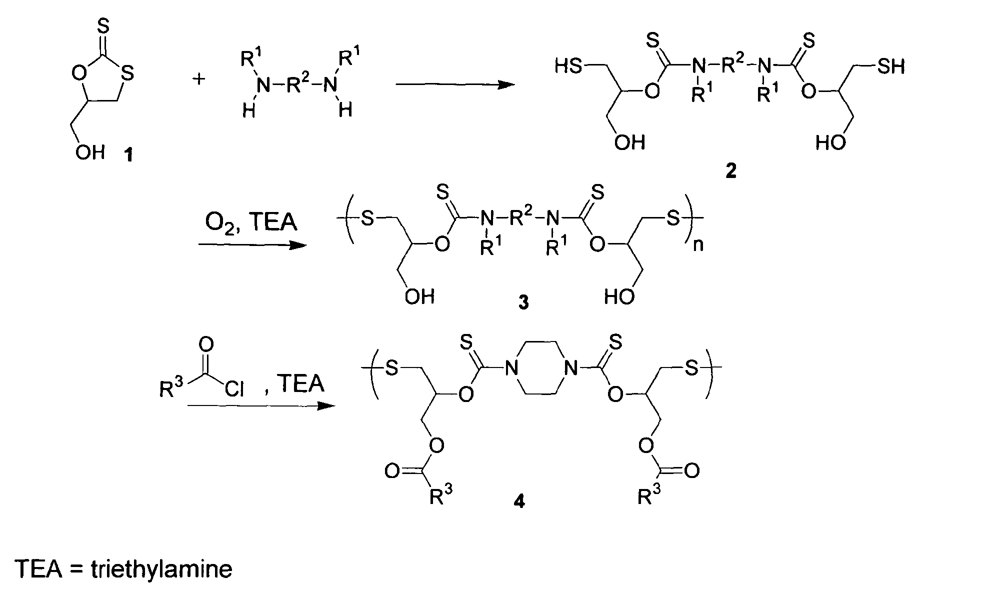

The precursor 2 is a useful component for adhesive, coating, and sealing materials. It has SH-groups, which can be used for rapid adhesion. The hydroxyl groups can react with epoxide, isocyanate etc.

The polymer 3 is a high sulfur-containing polymer. It has a high reflective index and can be used as an optical material. It also has a reactive hydroxyl group, which can be further used for functionalization such as cross-linking reaction by addition of diisocyanate, dicarboxylic chloride to obtain optical materials or can be used for coating or adhesion of other optical materials. Its reflective index can be controlled by choosing a suitable amine used for the polymer synthesis.

Since the polymer 3 has S-S and C=S bonds it is capable of capturing various inorganic salts and organometals of heavy metals such a Hg, Cr, Sn, Pb, etc. The hydroxyl groups of the polymer 3 can be used for cross-linking the polymer to obtain a corresponding membrane, which can be used as a filter to remove the above heavy metal compounds from wasted water.

Since the S-S bond is photo-cleavable, the polymer 3 and its derivatives can be used as photoresist materials. Photosensitivity can be controlled by modification of the hydroxyl groups. The thermal and mechanical properties of the resist material can be controlled by choosing a suitable amine.

The potency of DTC-OH compounds in the production of multifunctional monomers and polymers covers a wide range of products as can be seen from the following diagram. Especially, monomers having both DTC and isocyanate groups in one molecule can be successfully obtained, which is not attained by the conventional methods for DTC synthesis based on cycloaddition of epoxide and carbon disulfide, because isocyanate is damaged under such conditions.

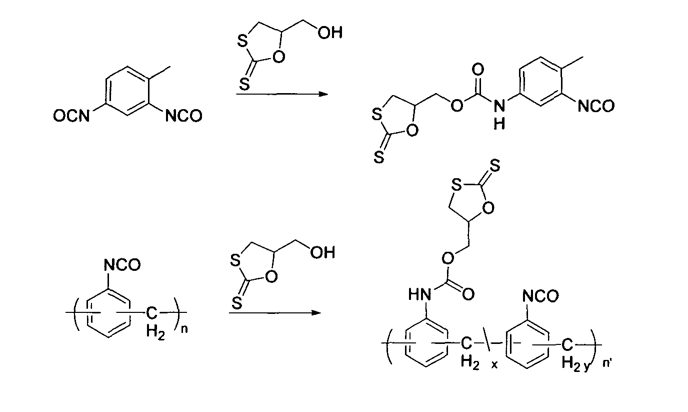

The DTC-isocyanate hybrid, which is also object of the invention can further be used as a precursor for various other multifunctional compounds. For example, addition of glycidol to the DTC-isocyanate hybrid gives a DTC-epoxy hybrid, which will be highly miscible in epoxy resin to afford high crosslinking degree. By choosing alcohol in the second step, properties of the compound such as melting point and viscosity, can be controlled. Suitable alcohols can be selected from aliphatic, cycloaliphatic, aromatic or araliphatic alkohols, which may carry further reactive groups and/or heteroatoms.

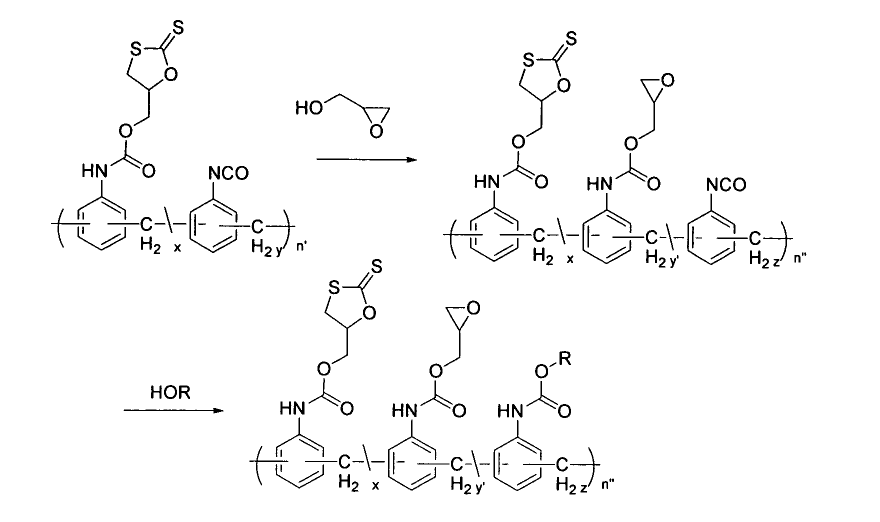

Beneath the above objects of the present invention, i.e. to provide a highly potent key intermediate, DTC-OH, and the thereof producible end products, as well as their applications and methods of production, the present invention further provides a process for producing the cyclic 5-memberd ring dithiocarbonate key intermediates having the general formula 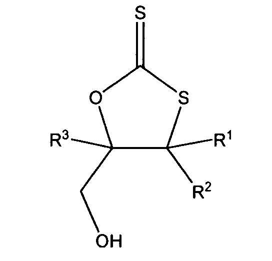 wherein, in the formula, R¹, R², and R³ are the same or different, each of which denotes hydrogen or a C1 to C4 straight-chain or branched alkyl which process comprises the steps of
a) reacting a compound with the following general formula 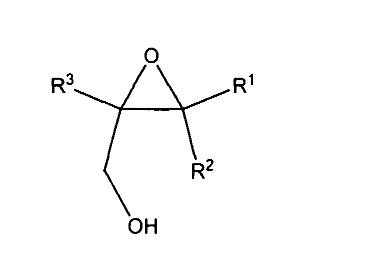 with an hydroxy group protecting agent to give the O-protected compound,
b) reacting the O-protected compound of step a) with carbon disulfide in the presence of a catalyst and
c) removing the protecting group by adding an aqueous acidic solution.

Particularly useful as protecting agent are trialkylsilyl halides, such as triethylsilyl chloride or t-butyldimethylsilyl chloride or silyl chloride residues in the side chain of polymers; or vinyl ethers of the following general formula R^{a}-CH=CH-OR^{b}, wherein R^{a} and R^{b} can be the same or different and denote a straight-chain or branched alkyl group with 1 to 12 carbon atoms or are part of the side chains of a polymer or R^{a} and R^{b} form a carbocyclic structure.

Preferably step a) of the process of the present invention is carried out without solvent or in an organic solvent in the present of an organic base. Suitable organic solvents are hydrocarbons such as heptane, hexane, benzene and toluene, ethers such as diethylether and tretrahydrofuran, esters such as ethyl acetate, amide such as N,N-dimethylformamide, N-methylpyrolidone, chlorinated hydrocarbons such as chloroform, dichloromethane or chlorobenzene.

Suitable organic bases are triethylamine, diisopropylethylamine, and pyridine. Polymer-supported amines such as (crosslinked) poly(vinylpyridine) and poly(4-(N,N-dimethylamino)methylstyrene) also can be used. Inorganic bases, which are a salts of strong base and a weak acid such as sodium carbonate, sodium bicarbonate, potassium carbonate, potassium bicarbonate, can be used. These inorganic bases can also be used after immobilization on other inert solids such as silicates, aluminium oxide and the like.

The preferred organic solvent is dichloromethane and the preferred organic base is an amine, preferably triethylamine.

Suitable catalyst for carrying out step b) of the process of this invention are Nal, Lil, LiBr or LiCl. The preferred catalyst is LiBr.

Embodiments of the present invention will be described by the following examples.

### PREPARATION EXAMPLES

### Synthesis of a dithiocarbonate compound having a hydroxyl group

The synthetic route is shown in Scheme 1. To a solution of glycidol (74 g, 1.0 mol) and triethylamine (110 g, 1.2 mol) in dichloromethane (650 ml), trimethylsilyl chloride (120 g) was added at 0 °C, and the resulting mixture was stirred at room temperature. After 17 h, the reaction mixture was washed with cold saturated aqueous NaHCO₃ solution and with brine, and was dried over Na₂SO₄. The mixture was filtered, concentrated under reduced pressure, and the residue was distilled in *vacuo* (38~40 mmHg, 63 °C) to give glycidyl trimethylsilyl ether (TMS-glycidol; 162 g, 92%).

To a solution of TMS-glycidol (14.6 g, 100 mmol) and LiBr (0.43 g, 5.0 mmol) in THF (100 ml), carbon disulfide (9.12 g, 120 mmol) was added dropwise at 0 °C, and the reaction mixture was stirred at room temperature. After 17 h, ethyl acetate (400 ml) was added, and the solution was washed with saturated aqueous NaCl three times to give a solution of the formed dithiocarbonate having silylether TMS-1. This solution was washed with cold aqueous 10% hydrochloric acid and with brine. Silyl group was removed by this operation. The organic layer was dried over Na₂SO₄, filtered, and concentrated under reduced pressure to give 5-hydroxymethyl-1,3-oxathiolane-2-thione (DTC-OH of Scheme 1) (9.75 g, 65 mmol, 65%).

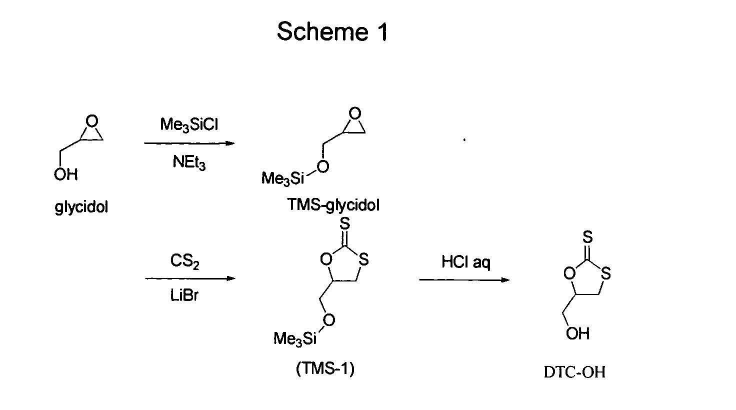

### Reaction of DTC-OH with diamines, subsequent oxidative polymerisation and modification

The synthetic route is shown in Scheme 2. To a solution of DTC-OH (0.33 g, 2.2 mmol) in THF (5 ml), piperazine (0.086 g, 1.0 mmol) was added at ambient temperature under nitrogen. After the resulting mixture was stirred for 24 h, triethylamine (TEA, 0.252 g, 2.5 mmol) was added to the mixture. The flask was flushed O₂, and the reaction mixture was stirred for 100 h under O₂ atmosphere (1 atm) at room temperature, to obtain the corresponding polythiourethane having hydroxyl pendant. The polymer having hydroxy terminal groups was soluble in methanol to indicate its hydrophilicity due to the hydroxyl pendant.
The solution of this "hydroxy polymer" was concentrated under reduced pressure, and the residue was suspended in CH₂Cl₂ (5 ml). To this suspension, a solution of TEA (0.44 g 4.4 mmol) in CH₂Cl₂ (5 ml) was added and cooled to below 10 °C. To the solution, a solution of acetyl chloride (0.346 g, 4.4 mmol) in CH₂Cl₂ (5 ml) was added dropwise. After the reaction mixture was stirred for 1 h, the reaction mixture became homogeneous. The reaction mixture was stirred for additional 5 h, and then was poured into brine. The organic phase was washed with brine three times, and the combined organic layers were dried over anhydrous sodium sulfate, filtered, and concentrated under reduced pressure. The residue was poured into a large amount of hexane, to give the corresponding "acetylated polymer" as precipitates.

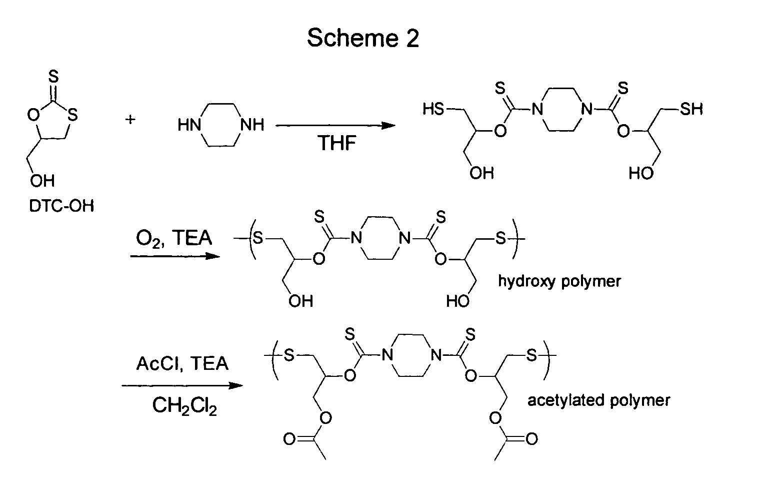

### Reactions of DTC-OH with various mono- and diisocyanates

### Reaction DTC-OH with o-, m- and p-tolylisocyanate

DTC-OH is reacted with tolylisocyanate smoothly in the presence of catalytic triethylamine as shown in Scheme 3. The reaction rate depends on the position of the methyl substituent on the benzene ring of the isocyanate as can be shown by time-conversion relationships, whereby the conversion time increases from ortho- to meta- to para-tolylisocyanate.

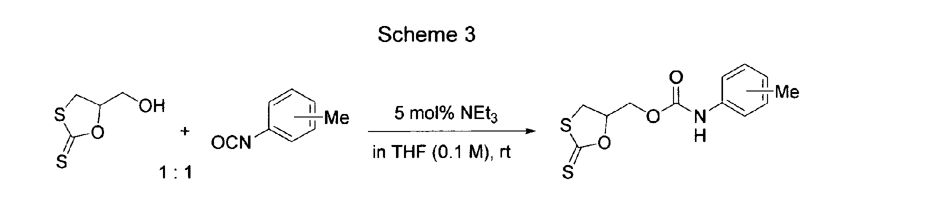

### Reactions of DTC-OH with other isocyanates

Reactions of DTC-OH with various isocyanates were carried out (Scheme 4). As a catalyst, tertiary amine or dibutyltin dilaurat was effective. The structures of the obtained products (DTC-urethanes) are listed in Table 1.

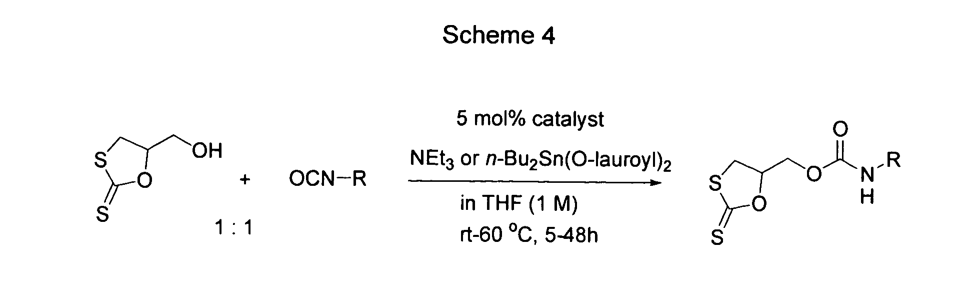

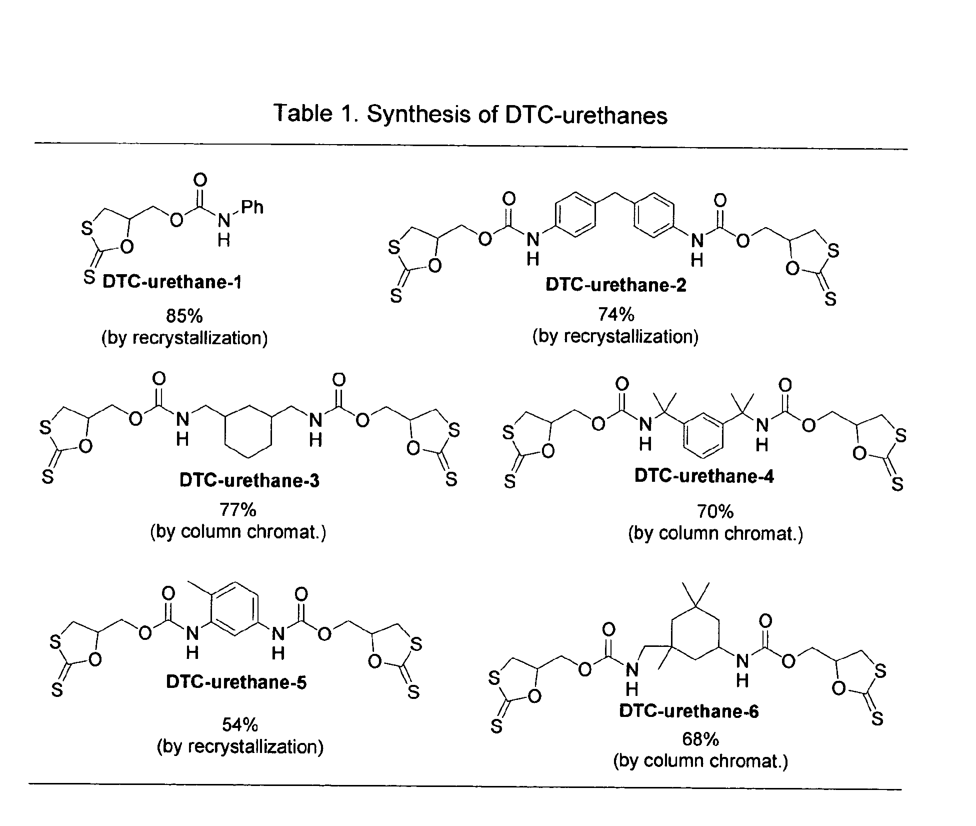

### Reactions of DTC-OH with polymers bearing isocyanate groups at their terminal positions

DTC-OH reacts with isocyanate groups at the terminal positions of various polymers, as e.g. shown in Scheme 5. Quantitative terminal functionalization was confirmed by IR analysis.

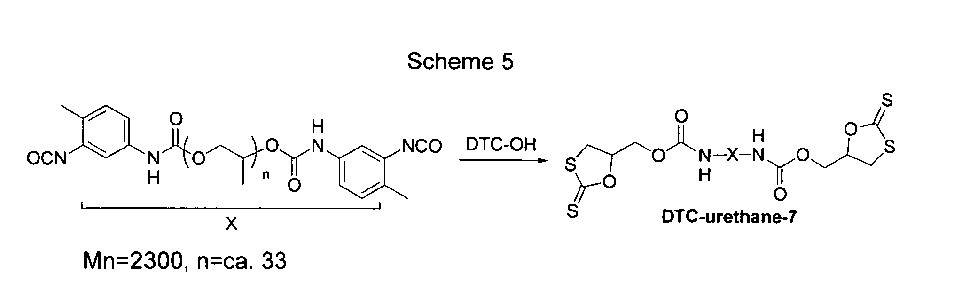

Polyurethanes having isocyanate groups at terminal positions can be similarly functionalized (Scheme 6). The obtained polymer can be crosslinked by various reactions of the dithiocarbonate unit.

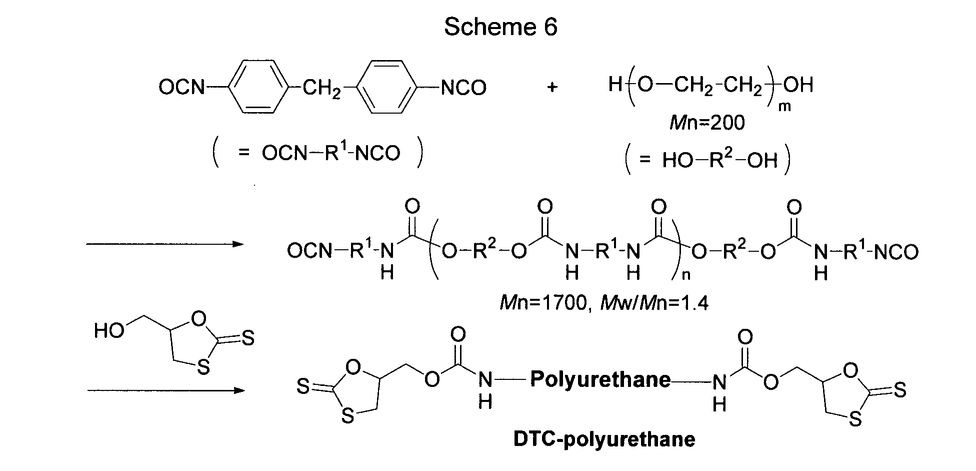

DTC-polyurethanes can also be synthesized by the following "one-step" method (Scheme 7). Since DTC group is highly reactive with amines and is polymerizable by cationic initiators, but not affected by moisture, the polymers having DTC groups at the terminal positions can be applied in a water-emulsion adhesives, paints, coatings and the like.

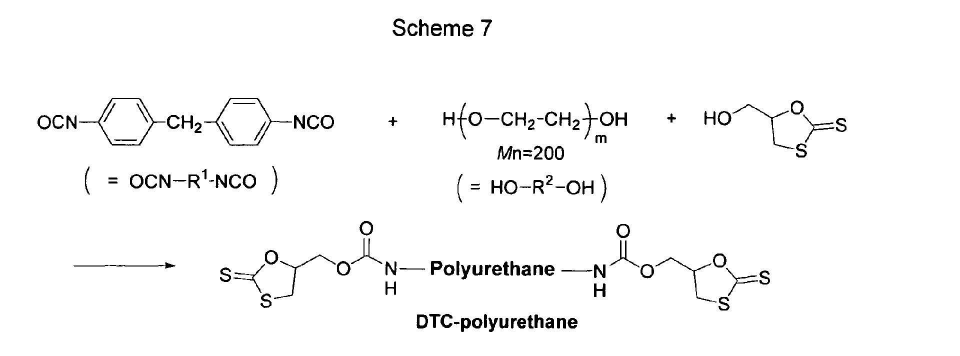

### Reactions of DTC-OH with polymers bearing isocyanate groups in the side chain of the polymer

DTC-OH reacts with isocyanate groups in the polymer side chain (Scheme 8). Quantitative terminal functionalization was confirmed by IR analysis.

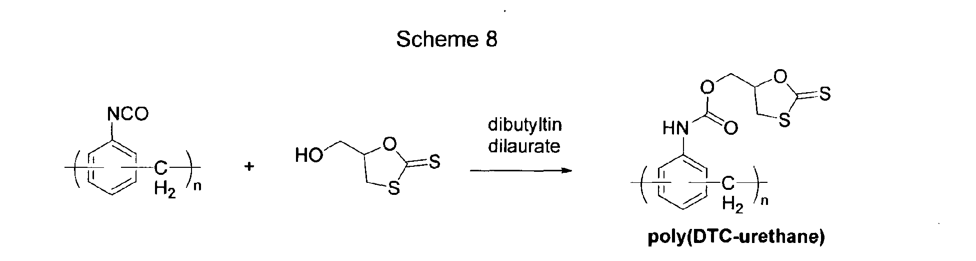

To a solution of poly(phenylisocyanate-co-formaldehyde) (PPFI; 4.0 g, 30.4 mmol NCO unit) and dibutyltin dilaurate (DBTDL, 0.96 g, 5 mol%) in dry tetrahydrofuran, a 20 ml of tetrahydrofuran solution of DTC-OH (5.0 g, 33.3 mmol) was added dropwise at 0 °C. After stirring the mixture at room temperature for 1 day, the crude product was reprecipitated from hexane to yield a pale yellow powder of PPIF-DTC. The reprecipitation process was repeated three times to remove the unreacted DTC-OH.

### Reactions of DTC-OH with carboxylic acid derivatives

### Benzoylation of DTC-OH

To a solution of DTC-OH (0.15g, 1.0 mmol) and triethylamine (0.12 g, 1.2 mmol) in dichloromethane (5.0 ml), a solution of benzoyl chloride (0.15 g, 1.1 mmol) in dichloromethane (10 ml) was added dropwise at 0 °C, then the mixture was stirred at room temperature. After 7 h, the mixture was washed with saturated aqueous solution of sodium chloride. The dichloromethane layer was dried with sodium sulfate, filtered and was concentrated under reduced pressure. The residue was fractionated by column chromatography (silica gel) to obtain the corresponding ester (0.25 g, 1.0 mmol) quantitatively.

### Methacryloylation of DTC-OH

DTCOMA was similarly synthesized by esterification with methacryloyl chloride.

### Reaction of DTC-OH with diketene

To a mixture of DTC-OH (9.28 g, 61.8 mmol) and triethylamine (0.0691 g, 6.89 mmol), diketene (6.43 g, 76.4 mmol) was added over a period of 1 h at room temperature, and the mixture was stirred at the same temperature for 2 h. After removal of volatiles under reduced pressure, the residue was fractionated by column chromatography (silica gel, eluent: hexane / ethyl acetate = 1 /1) to obtain DTC-acac (13.2 g, 56.5 mmol, 91%) as a pale yellow oil: ¹H-NMR (CDCl₃, 20 °C) 5.36-5.30 (m, 1 H, -CH₂-C*H*(-O-)-CH₂-), 4.55-4.45 (two dd, 2H, -CH(-O-)-C*H*_{*2*}-S-), 3.70-3.59 (two dd, 2H, -CH(-O-)-C*H*_{*2*}*-*O-), 3.57 (s, 2H, -C(=O)-C*H*_{*2*}-C(=O)-), 2.28 (s, 3H, -C(=O)-C*H*_{*3*}) ppm; ¹³C-NMR (CDCl₃, 20 °C) 211.2 (-S-*C*(=S)-O-), 200.2 (-CH₂-*C*(=O)-CH₃), 166.3 (-O-*C*(=O)-CH₂-) 87.2 (-CH₂-*C*H(-O-)-CH₂-), 63.0 (-CH(-O-)-*C*H₂-O-), 49.3 (-C(=O)-*C*H₂-C(=O)-), 35.5 (-S-*C*H₂-CH(-O-)-), 30.1 (-C(=O)-*C*H₃) ppm; IR (neat) 3002, 2953, 1747, 1715, 1650, 1557, 1439, 1410, 1359, 1316, 1233, 1190, 1148, 1043, 918, 848 cm⁻¹.

### APPLICATION EXAMPLES

### Application of the derivative monomers as a component of an epoxy formulation

As components of a standard formulation, which is representative for various adhesive and sealant applications, bisphenol A diglycidyl ether (Bis A-DGE) and polypropyleneglycol having amino terminals were chosen. In some cases, glycidyl phenyl ether was added to decrease the viscosity of the mixture for an easy handling. Different cyclic dithiocarbonate compounds (DTCs), 0.15 equivalent of epoxy group, were added as a third component. The initial amount of amino group was set to be equal to [epoxy group]₀/2+[DTC]. Bis A-DGE and DTC were mixed with stirring and with evacuation to degas. If DTC does not dissolve in Bis A-DGE, the mixture was heated (maximum 160 °C) until the mixture became homogeneous. The mixture was cooled to 0 °C, and then Jeff-amine was added to the mixture, with stirring under vaccum. Then, 20 ml of the mixture was weighted in a volume measuring flask, to calculate the density. The mixture was transferred to a mold (6.0 mm*60mm*70mm) and cured (at 120 °C for 1 h, or at 50 °C for 24h) to obtain the corresponding cured resin plate, of which density was measured by electronic densimeter. Degree of volume change (V _{after curing}-V _{before curing})/(V _{before curing}) was calculated from the density values by the equation (D _{before curing})/(D _{after curing})-1.

### A typical procedure is given for the case using DTC-urethane-2

BisA-DGE (15.24 g, 44.8 mmol, amount of epoxy group=89.5 mmol) and DTC-urethane-2 (4.62 g, 8.39 mmol, amount of DTC group=16.79) was mixed with stirring and with evacuation. The mixture was heated at 160 °C until it became homogeneous (approximately 10 min). After cooling to room temperature, glycidyl phenyl ether (GPE, 3.36 g, 22.4 mmol) was added to the mixture, and stirred to obtain a homogeneous mixture. Jeff-amine (pre-cooled at 0 °C; 14.5 g, amount amino group=72.7 mmol) was added to the epoxy-DTC mixture and was mixed with degassing under vacuum below 15 °C for 30 min. The obtained mixture was transferred into a 20 ml volume measuring flask. Based on the weight of 20 ml of the mixture, the density of the epoxy formulation *before* curing was calculated. The density was (22.10 g)/(20.00 ml)=1.105. Then, 24.37 g of the mixture was transferred into a silicone mold, and was cured at 50 °C for 24 h, to obtain 24.37 g of the plate-shaped cured resin. Its density was measured by electronic densimeter to be 1.158. Based on the density values, the degree of volume change was calculated to be -4.6%.

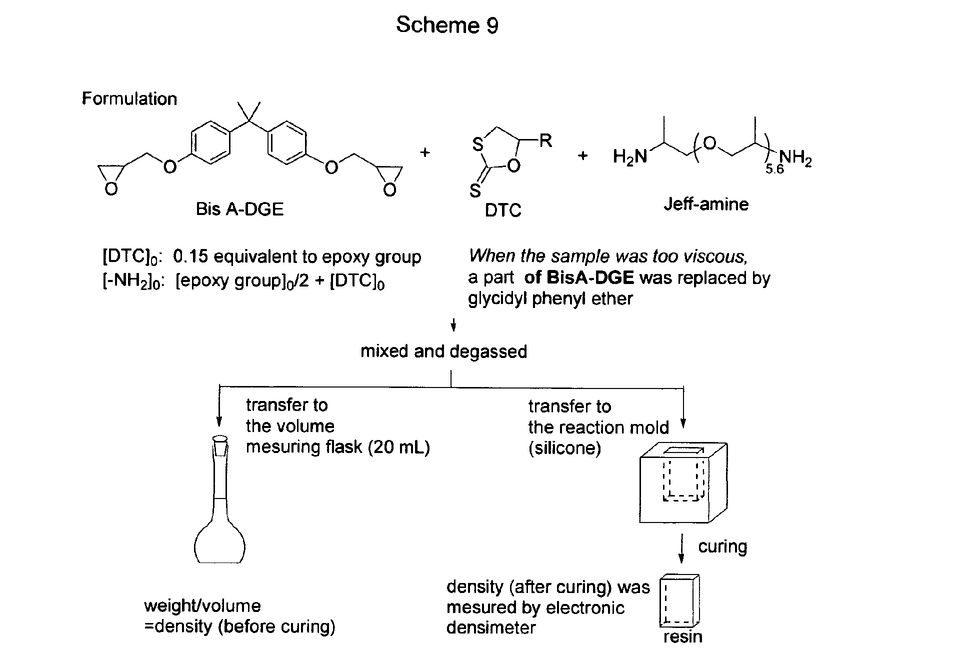

In Table 2, the examined formulations and the corresponding volume change degrees are listed. Most of the DTCs are effective to suppress the volume shrinkage. The known dithiocarbonates: 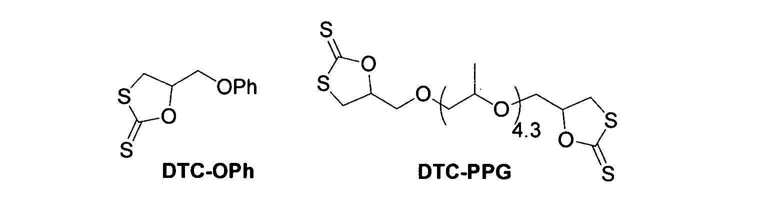 were also examined. When the curing reaction was carried out without dithiocarbonate (DTC), 5.6-6.6% volume shrinkage was observed. On the other hand, if the curing reaction was carried out in the presence of DTC, the volume shrinkage was reduced to be below 5%. Especially, DTCs having a urethane moiety, which were synthesized by the reaction of DTC-OH and isocyanates, were effective.

**Table 2.**

| Volume change of the curing reactions | | | | | |
|---|---|---|---|---|---|
| DTC | temp | GPE | density (g/ml) | | volume change |
| | / °C | content | before | after | |
| none | 120 | | 1.080 | 1.150 | -6.1% |
| none | 120 | | 1.078 | 1.143 | -5.7% |
| none | 120 | 5% | 1.074 | 1.146 | -6.3% |
| none | 120 | 10% | 1.074 | 1.145 | -6.2% |
| none | 120 | 20% | 1.071 | 1.138 | -5.9% |
| none | 120 | 30% | 1.060 | 1.135 | -6.6% |
| none | 50 | | 1.070 | 1.135 | -5.7% |
| none | 50 | | 1.080 | 1.143 | -5.5% |
| none | 50 | 20% | 1.075 | 1.139 | -5.6% |
| DTC-OPh | 120 | | 1.080 | 1.150 | -6.1 % |
| DTC-OPh | 50 | | 1.091 | 1.152 | -5.3% |
| DTC-PPG | 120 | | 1.090 | 1.150 | -5.2% |
| DTCOBz | 120 | | 1.080 | 1.150 | -6.1% |
| DTCOBz | 50 | | 1.094 | 1.151 | -5.0% |
| DTCOMA | 120 | | 1.090 | 1.145 | -4.8% |
| DTC-urethane-1 | 50 | | 1.090 | 1.140 | -4.4% |
| DTC-urethane-2 | 50 | 20% | 1.105 | 1.158 | -4.6% |
| DTC-urethane-3 | 50 | | 1.100 | 1.158 | -5.0% |
| DTC-urethane-4 | 50 | | 1.100 | 1.155 | -4.8% |
| DTC-urethane-5 | 120 | 30% | 1.086 | 1.135 | -4.3% |
| DTC-urethane-5 | 50 | | 1.100 | 1.155 | -4.8% |
| DTC-urethane-5 | 50 | 20% | 1.095 | 1.148 | -4.6% |
| DTC-urethane-7 | 50 | | 1.086 | 1.144 | -5.1% |
| poly(DTC-urethane) 5% | 120 | 20% | | | -5.8 |
| poly(DTC-urethane) 10% | 120 | 20% | | | -5.2 |
| poly(DTC-urethane) 15% | 120 | 20% | | | -4.4 |

The addition of DTC was effective not only to reduce the volume shrinkage but also to enhance adhesion strength as will be shown below.

### Adhesion test

The adhesion test was performed according to the "ASTM D1002 Apparent Shear Strength of Single-Lap-Joint Adhesively Bonded Metal Specimens by Tension Loading (Metal-to-Metal)" method, using a tension testing machine (model: 4204, manufactured by INSTRON, U.S.A.) and as specimens grid blasted mild steel lapshear specimens (25.4*101.6*1.6 mm (TS101)). The test was performed at 23 °C under an atmosphere of 50% relative humidity. The samples were identical to those used for the volume-change studies (cured at 50 °C for 24 h). Table 3 summarizes the results of the adhesion test.

**Table 3.**

| Adhesion test | | |
|---|---|---|
| DTC | shear strength in N/mm² | tensile force in kN |
| none | 23.7 | 7.51 |
| DTC-OPh | 21.9 | 6.96 |
| DTC-urethane-3 | 27.4 | 8.55 |
| DTC-urethane-4 | 25.5 | 7.97 |

## Claims

1. Cyclic 5-membered ring dithiocarbonate compounds of the general formula 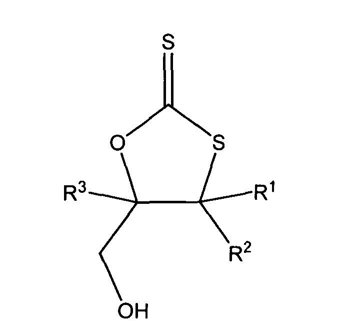 wherein, in the formula, R¹, R², and R³ are the same or different, each of which denotes hydrogen or a C1 to C4 straight-chain or branched alkyl.

2. The compounds of claim 1 wherein R¹, R², and R³ denote hydrogen.

3. A process for producing the cyclic 5-membered ring dithiocarbonate compounds of claim 1 having the general formula 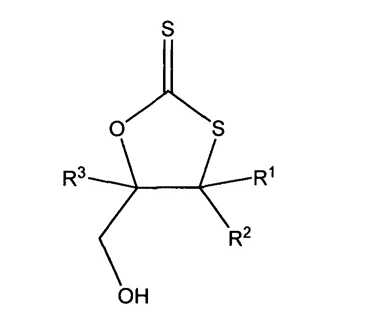 wherein, in the formula, R¹, R², and R³ are the same or different, each of which denotes hydrogen or a C1 to C4 straight-chain or branched alkyl which process comprises the steps of
c) reacting a compound with the following general formula 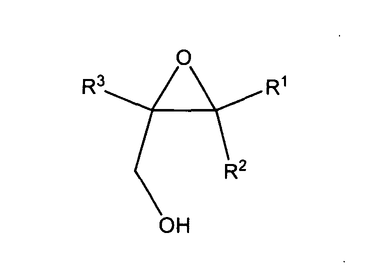 with an hydroxy group protecting agent to give the O-protected compound,
d) reacting the O-protected compound of step a) with carbon disulfide in the presence of a catalyst and
c) removing the protecting group by adding an aqueous acidic solution.

4. A process according to claim 3, wherein the protecting agent is a trialkylsilyl halide or a vinyl ether of the following general formula R^{a}-CH=CH-OR^{b}, wherein R^{a} and R^{b} can be the same or different and denote a straight-chain or branched alkyl group with 1 to 12 carbon atoms or are part of the side chains of a polymer or R^{a} and R^{b} form a carbocyclic structure.

5. A process according to claim 4, wherein the protecting agent is a triethylsilyl chloride or a t-butyldimethylsilyl chloride or a silyl chloride residue in the side chain of a polymer.

6. The process of claim 3, wherein step a) is carried out in an organic solvent in the presence of an organic base or an organic acid.

7. The process of claim 6, wherein the organic solvent is dichloromethane and the organic base is an amine.

8. The process of claim 3 to 7 wherein the catalyst of step b) is Nal, Lil, LiBr or LiCl.

9. A linear polymer having thiourethane and disulfide bonds in the main chain and hydroxy groups in the side chains obtainable by reacting the cyclic 5-membered ring dithiocarbonate compounds of claim 1 having the general formula 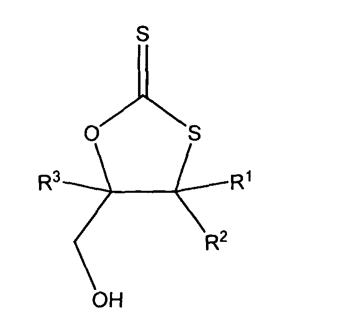 wherein, in the formula, R¹, R², and R³ are the same or different, each of which denotes hydrogen or a C1 to C4 straight-chain or branched alkyl, with a secondary diamine in an organic solvent and reacting the resultant intermediate compound with oxygen.

10. The polymer of claim 9, wherein the pendant hydroxyl groups of the side chains in a further step are reacted with acyl halides, acid anhydrides, isocyanate, vinyl ether, or silyl chloride.

11. A process for producing 5-membered cyclic dithiocarbonate compounds having ester group by reacting a compound according to claim 1 or 2 with a mono-, di- or poly-carboxylic acid or mono-, di- or poly-carboxylic acid derivative selected from the group consisting of "carboxylic acid anhydrides, lactones, activated carboxylic esters and carboxylic acid halides ".

12. The compound obtainable by the process of claim 11, wherein the carboxylic acid derivative is a cyclic carboxylic acid anhydride or lactone.

13. The compound obtainable by the process of claim 11, wherein the dicarboxylic acid derivative is a polyester with activated carboxylic acid groups at both ends of the polyester chain.

14. A process for producing the compound of claim 13, whereby the polyester with activated carboxylic acid groups at both ends of the polyester chain is formed in the presence of the compound according to claim 1 or 2.

15. A process for producing 5-membered cyclic dithiocarbonate compounds having one or more urethane groups by reacting a compound according to claim 1 or 2 with an isocyanate.

16. The compound obtainable by the process of claim 15, wherein the isocyanate is a mono- or oligomeric monoisocyanate or a polymer having one isocyanate group at the chain end.

17. The compound obtainable by the process of claim 15, wherein the isocyanate is a polymer having isocyanate groups in the side chain.

18. The compound obtainable by the process of the claim 15, wherein the isocyanate is a polymer with isocyanate groups at both ends of the polymer chain.

19. The compound obtainable by the process of the claim 15, wherein the isocyanate has the general formula: 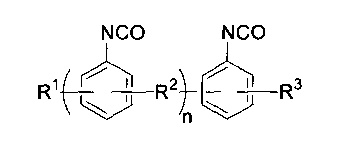 wherein,
n>0;
the benzene rings are optionally substituted by further reactive and/or non-reactive functional groups and/or fused with a further aromatic ring;
R¹, R² and R³ can be attached at any position of the benzene ring or fused benzene ring;
R¹ and R³ can be same or different and denote hydrogen, (hetero)alkyl, (hetero)aromatic and halogen; and
R² is a (hetero)alkylene residue, a (hetero)aromatic residue, oxygen, sulfur, oxidized sulfur, a carbonyl residue, a substituted silicone residue, or any polymer chain.

20. The compound of claim 17 or 18, wherein in average only one group of the diisocyanate is reacted with the compound of claim 1 or 2.

21. The compound of claim 19, wherein the isocyanate groups are only partially reacted with the compound of claim 1 or 2.

22. The compound of claim 21, wherein the excessive isocyanate groups are further reacted with at least one hydroxy functional compound.

23. The compound of claim 22, wherein the hydroxy functional compound is glycidol and/or an aliphatic, cycloaliphatic, aromatic or araliphatic alkohol, optionally further reactive groups and/or heteroatoms.

24. Use of any of the compounds of claims 1 and 2, the compounds obtainable by the process of claim 11, the compounds of claims 12 and 13, the compounds obtainable by the process of claim 15, the compounds of claims 16 to 23, or 9 before and after carrying out the oxidation step, in adhesive, coating or sealing compositions.

25. The use according to claim 24, wherein the composition is an epoxy resin composition.

26. The use of any of compounds of claims 9 and 10 in the manufacture of optical and/or photoresist materials.
